(19) Europäisches Patentamt
European Patent Office
Office européen des brevets

(11) Publication number: **0 292 128**
**A1**

(12) **EUROPEAN PATENT APPLICATION**

(21) Application number: **88303847.3**

(51) Int. Cl.⁴: **C12Q 1/68**

(22) Date of filing: **28.04.88**

(30) Priority: **28.04.87 US 43529**

(43) Date of publication of application:
**23.11.88 Bulletin 88/47**

(84) Designated Contracting States:
**AT BE CH DE ES FR GB GR IT LI LU NL SE**

(71) Applicant: **TAMIR BIOTECHNOLOGY LTD**
**POB 3702**
**Petach Tikva(IL)**

(72) Inventor: **Segev, David**
**64 Amsterdam Avenue**
**Passaic NJ 07055(US)**

(74) Representative: **Ruffles, Graham Keith et al**
**MARKS & CLERK 57-60 Lincoln's Inn Fields**
**London WC2A 3LS(GB)**

(54) **Improved DNA probes.**

(57) Labeled DNA molecules are provided which can be used as highly sensitive molecular probes for the detection of specific RNA or DNA molecules. These DNA probes comprise hybridizable oligonucleotides complementary to the specific RNA or DNA molecules which are covalently coupled through their 5' hydroxyl groups to novel labeling arrays. These arrays contain branched networks bearing terminal reporter groups which can be detected by direct or indirect means.

EP 0 292 128 A1

# IMPROVED DNA PROBES

## TECHNICAL FIELD

This invention relates to single-stranded DNA molecules labeled at the 5′ termini with a plurality of reporter groups, which are useful as highly sensitive molecular probes for nucleic acid hybridization or in situ gene localization.

## BACKGROUND OF THE INVENTION

Labeled DNA molecules have become one of the most important tools in modern cellular and molecular biology. Through the use of such labeled molecules, which are often referred to as molecular probes, it has been possible to locate specific genes in genomic libraries, to study the mechanisms of gene expression and oncogene amplification, to localize specific genes to individual chromosomes and to detect infectious agents such as viruses in biological fluids and tissue samples.

Typically, a synthetic oligonucleotide or natural DNA strand is labeled with a molecule, known as a reporter molecule, which facilitates detection of the DNA molecule after it has specifically bound to complementary sequences in a DNA or RNA molecule of interest. A wide range of reporter molecules has been used, both radioactive and non-radioactive.

The recent trend has been to use non-radioactive labeling methods, both to eliminate hazards to personnel and the environment inherent in the use of radioisotopes and to produce increased sensitivity of detection. Most commonly, fluorescent molecule or biotin labeling is used. Biotin labeling can be detected through the use of avidin or streptavidin labeled, e.g., with an enzyme such as alkaline phosphatase or a peroxidase. Alternatively, the biotin labels can be detected using suitably labeled antibodies against biotin. This approach has also been used with other haptenic labels such as 2,4-dinitrobenzene.

The use of enzyme-labeled avidin or antibodies to detect biotin labeled DNA produces greatly increased sensitivity, since each enzyme molecule can convert many molecules of an appropriate substrate to a readily detectable product. The sensitivity of the method, however, is limited by the number of biotin molecules that can be introduced into a DNA strand without impairing the hydridization process.

Various methods have been developed for introducing multiple biotin molecules into DNA. For example, Langer et al. [Proc. Natl. Acad. Sci. USA 78:6633 (1981)] and Singer et al. [Proc. Natl. Acad. Sci. USA 79:7331 (1982)] have prepared biotinylated analogs of dUTP, and then incorporated these molecules into DNA by nick-translation. In these analogs, biotin is covalently coupled to the pyrimidine ring via linker molecules.

Similar methods have been described by Leary et al. [Proc. Natl. Acad. Sci. USA 80:4045 (1983)] and Langer-Safer et al. [Proc. Natl. Acad. Sci. USA 79:4381 (1982)] using biotinylated analogs of dUTP. Landes [U.S. patent No. 4,626,501] has described a similar method for the labeling of adenine and cytosine bases in DNA molecules.

Unfortunately, because these labels modify the base rings of the nucleotides and are introduced throughout the DNA molecules at points where hydridization occurs, the number of molecules that can be incorporated must be limited. Otherwise, the melting temperature (Tm) of hybridization complexes formed using the labeled DNA will be substantially lowered due to steric hindrance by the label molecules.

In an effort to reduce the steric hindrance problem Landes, supra, has disclosed methods by which adenosine and cytosine bases are labeled in a homo- or heteropolymeric tail portion which is attached to the unlabeled hydridizing part of a DNA probe. This labeling method also entails nucleotide base ring modification.

## SUMMARY OF THE INVENTION

Methods and compositions are provided for the detection of specific RNA or DNA sequences in biological materials. The compositions of the invention are labeled, single-stranded DNA molecules, comprising:

(a) an oligonucleotide complementary to a specific RNA or DNA sequence which is capable of specifically hybridizing thereto; and

(b) a labeling array covalently coupled to the 5′ terminal hydroxyl group of the oligonucleotide which comprises in order from such hydroxyl group

(i) a first linker molecule of the formula

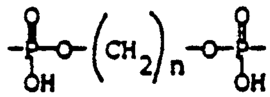 or 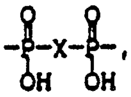

where n = a number from 2 to 10 and X represents a polyethylene glycol chain having from 1 to 30 ethylene glycol units;

(ii) a polyhydroxyl branching molecule having from 3 to 5 hydroxyl groups, one of which is covalently coupled to the first linker molecule through its distal phosphate group; and

(iii) second linker molecules of the formula

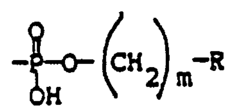 or 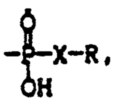

where m = a number from 2 to 10, X represents a polyethylene glycol chain having from 1 to 30 ethylene glycol units, and R is selected from the group consisting of a reporter group; one or more additional first linker molecules terminated by a reporter group; or one or more labeling arrays comprising such first linker molecules, polyhydroxy branching molecules and second linker molecules, the ends distal to the oligonucleotide of which are terminated by reporter groups;

which second linker molecules are covalently coupled by the phosphate groups to the other hydroxyl groups of such branching molecule.

This invention also provides methods for detecting specific RNA or DNA sequences in biological samples, comprising:

(a) contacting a biological sample suspected to contain a specific RNA or DNA sequence under conditions which promote specific hybridization of complementary nucleotide sequences with a labeled single-stranded DNA molecule, comprising:

(i) an oligonucleotide complementary to the specific RNA or DNA sequence which is capable of specifically hydridizing thereto; and

(ii) a labeling array covalently coupled to the 5′ terminal hydroxyl group of the oligonucleotide which comprises in order from such hydroxyl group

(A) a first linker molecule of the formula

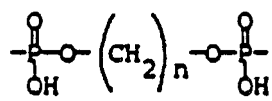 or 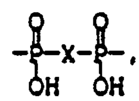

where n = a number from 2 to 10 and X represents a polyethylene glycol chain having from 1 to 30 ethylene glycol units;

(B) a polyhydroxyl branching molecule having from 3 to 5 hydroxyl groups, one of which is covalently coupled to the first linker molecule through its distal phosphate group; and

(C) second linker molecules of the formula

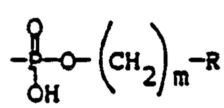 or 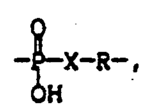

where m = a number from 2 to 10, X represents a polyethylene glycol chain having from 1 to 30 ethylene glycol units, and R is selected from the group consisting of a reporter group; one or more additional first linker molecules terminated by a reporter group; or one or more labeling arrays comprising such first linker molecules, polyhydroxy branching molecules and second linker molecules, the ends distal to the oligonucleotide of which are terminated by reporter groups;

which second linker molecules are covalently coupled by the phosphate groups to the other hydroxyl

groups of such branching molecule; and

(b) detecting any hydridization which occurs between such labeled DNA molecule and specific RNA or DNA sequence in the biological sample.

## BRIEF DESCRIPTION OF THE FIGURES

The present invention may be more readily understood by reference to the following figures, in which

Fig. 1 is a schematic representation of various labeled DNA probes of the invention in which

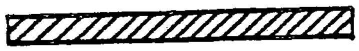

represents a hybridizable oligonucleotide or oligodeoxynucleotide specific for part of an RNA or DNA molecule that is to be detected, P represents a phosphate group,



represents a linker molecule, 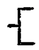 represents a polyhydroxyl branching molecule, and R represents a reporter molecule; and

Figs. 2-4 show the nmr spectra for the o-methyl N,N-di-phosphoramidite of di-p-methoxytritylhexaethyleneglycol; the o-methyl N,N-diisopropylphosphoramidite of the 1,7-di-p-methoxytrityl ether of 1,4,7-heptanetriol and the o-methyl N,N-diisopropylphosphoramidite of 6-trifluoroacetamidohexanol, respectively.

## DESCRIPTION OF THE INVENTION

As used herein, the term "reporter group" means a directly or indirectly detectable label group. Directly detectable groups that can be used include, e.g., radioisotopes such as $^{3}H$, $^{14}C$, $^{35}S$ and $^{125}I$, which can be readily incorporated into the molecular probes of the invention in many forms well known in the art. Ready incorporation of such radioisotopes into the probes is possible due to the availability of amino, carboxyl, sulfhydryl, maleimide, isocyanate, isothiocyanate or other groups that can be coupled to the termini of the probes distal to the hybridizable DNA segments. The isocyanate and isothiocyanate derivatives can be prepared by treating amino-terminal probe derivaties with phosgene and thiophosgene, respectively.

Other directly detectable reporter groups that can be employed include chemiluminescent, bioluminescent or fluorescent molecules such as fluorescein or isoluminol; molecules that are opaque in the electron microscope (electron opaque) such as ferritin; or enzymes such as various peroxidases, glucose oxidase, β-galactosidase and alkaline phosphatase, which can be detected by their activity on appropriate substrate molecules. Horseradish peroxidase, which can be detected by spectrophotometric analysis of its activity on a substrate such as pyrogallol or o-phenylenediamine, is especially preferred. When enzymes are used, the probes can be used in conjunction with conventional additives, buffers, diluents and enzyme stabilizers.

The modified DNA molecules of the invention can also be unlabeled, indirectly detectable first members of specific binding pairs, which first members are detectable by addition to hybridization complexes containing such modified DNA molecules of labeled, second members of such binding pairs, or unlabeled second members of such binding pairs followed by labeled molecules specific for such second members. For example, biotin molecules which can later be detected by adding a molecule with specific affinity for biotin, such as avidin or streptavidin which is labeled as described above, can be used in the probes. Alternatively, a hapten molecule such as dinitrophenol can be added to the termini of the probes. Such hapten molecules can then be detected by the addition following hybridization of a labeled, specific antibody against the hapten molecules. This kind of detection could also be carried out by adding a first, unlabeled antibody from one animal species specific for the hapten, followed by a second, labeled antibody from another animal species which is specific for an immunoglobulin chain of the first antibody, etc.

The molecular probes of this invention comprise hydridizable oligonucleotide or oligodeoxynucleotide sequences that are complementary to part of the nucleic acid sequences of RNA or DNA molecules of interest, and various linker molecules and polyhydroxyl branching molecules which are covalently coupled to the hybridizable oligonucleotide sequences to produce labeling arrays having various configurations.

The basic linker molecules are preferably alkyl chains having from about 2 to 10 methylene groups, or polyethylene glycol chains having from 1 to about 30, preferably from about 4 to 6 ethylene glycol units,

which basic linker molecules are terminated at both ends by phosphate groups. As used in this invention, the term "ethylene glycol unit" means the group $-CH_2CH_2-O-$, which is repeated in the linker molecules. Alkyl chains that could be used preferably include ethyl, propyl, butyl, etc. chains up to decyl chains. Longer chains could be used, although hydrophobicity might then become a problem.

The exact nature of the linker molecules is not critical to the invention, as long as the atoms comprising them confer useable water solubility properties on the finished probes. Other chains incorporating sulfur, nitrogen, carbon and oxygen atoms, or combinations thereof, can be used as well as alkyl or polyethylene glycol chains. Moreover, these basic linker molecules can be omitted, with branching molecules attached directly to the 5′ positions of the oligonucleotides.

Other linker molecules, which are called terminal linker molecules, are used to terminate the parts of the probes that are distal from the hybridizable oligonucleotide sequences. These terminal linker molecules are similar to the basic linker molecules of the invention, except that they are terminated by a group that can be used to couple a suitable reporter group. These end groups can be, e.g., carboxyl, sulfhydryl, maleimide, isocyanate, isothiocyanate or, preferably, amino groups.

Used in conjunction with the basic and terminal linker molecules are polyhydroxyl branching molecules having preferably from 3 to 5 hydroxyl groups. The function of these branching molecules is to combine the various linker molecules into labeling arrays of varying branched configurations. The branching molecules can be, for example, molecules such as carbohydrates (e.g., glucose, mannose, fructose, sucrose, etc.) and derivatives thereof; glycerol or derivatives thereof and aromatic ring compounds substituted with hydroxyl-terminal alkyl, alkenyl or alkynyl chains. Heptanetriol branching molecules are preferred because they provide a convenient means for providing two attachment sites on a basic linker molecule. If linker molecules containing more than 5 hydroxyl groups are used, there may be problems of steric hindrance.

In the construction of the molecular probes of this invention, the requisite hybridizable oligonucleotide is preferably synthesized using standard solid state methods known in the art. While the oligonucleotide is still attached to its solid support, the components of the labeling arrays are conveniently assembled.

Construction of the labeling arrays proceeds by attachment of one or more basic linker molecules to the 5′ hydroxyl group of the immobilized oligonucleotide, followed by the attachment of the polyhydroxyl branching molecules, further basic linker molecules etc. until the desired degree of chain branching and elongation is achieved. Once the desired labeling array configuration has been produced, terminal linker molecules are attached with appropriate end groups, and reporter groups are attached to these end groups. The construction of the labeling arrays is preferably carried out using activated, phosphoramidite derivatives of the synthetic components, as described in the examples below.

Preferably, the labeling arrays are constructed in a symmetrical fashion, using linker molecules of equal length for a given stage of the synthesis. Asymmetric arrays, however, could also be produced. Some of the possible labeling arrays are shown schematically in Fig. 1.

In principle, there is no limit to the number of reporter groups that can be attached to the molecular probes of the invention. In general, an array bearing $2^n$ (where n is a positive integer) terminal reporter groups can be constructed using $2^n-1$ heptanetriol branching molecules in combination with various linker molecules. As a practical matter, however, steric hindrance may become a problem with very large labeling arrays. Thus, molecular probes containing from 2 to 8 reporter groups are preferred.

The advantage of larger labeling arrays lies in the fact that as the number of reporter groups in a molecular probe is increased, the detection signal strength increases accordingly. As used in this context, the term "signal strength" means the intensity of the event used for detection, such as emission of radioactivity or luminescence, conversion of a substrate to a product by an enzyme, etc. Increased signal strength may also be produced by using a number of probes having a smaller number of reporter molecules, each of which has a hybridizable oligonucleotide segment complementary to a different region of the RNA or DNA that is to be detected. By using mixtures of such probes for hybridization, multiple binding of the probes along different regions of each target molecule will occur, with a greatly increased overall signal strength.

The molecular probes of this invention can be used in any application in which conventional probes can be used. For example, they can be used to detect and/or quantify DNA or RNA immobilized on filters or to hybridize to nitrocellulose filters containing replicas of bacteriophage plaques or bacterial colonies. The probes can also be used for RNA or DNA detection or for in situ localization of genes in human or animal blood, serum or tissue samples. The conditions for use of the probes in hybridization assays should be stringent, i.e., they should be such that non-specific binding of the probes is minimized.

Appropriate hybridization conditions can be found in the Examples below and in references such as Grunstein et al., Proc. Natl. Acad. Sci. USA 72:3961 (1975); Falkow et al. U.S. patent No. 4,358,535; and Maniatis et al., Molecular Cloning: A laboratory Manual, Cold Spring Harbor Laboratory, pp. 309-361, 382-389 (1982), which are hereby incorporated by reference. Following washing procedures to remove free probe molecules, the quantity of specifically hybridized probe can be determined.

Generally, after hybridization has been carried out, the probes in the hybridization complexes are detected and/or quantified using a technique appropriate for the reporter groups used. If the reporter groups are directly detectable, the hybridization complexes are simply detected using an appropriate means, e.g., by detecting fluorescence, light emission, radioactivity or electron density.

If the reporter groups are only indirectly detectable and must be complexed with one or more other molecules for detection, then a step of contacting the hybridization complexes with the other molecule(s) that is detectable must first be carried out. For example, if the reporter groups are members of a specific binding pair, e.g., the antigen (hapten) of an immunological pair, the complexes are contacted with one or more antibodies, one of which is appropriately labeled. If the reporter group is a biotin molecule, the hybrid complexes can be contacted with labeled avidin for detection. Alternatively, because avidin is a glycoprotein consisting of four identical subunits, each of which can bind biotin, unlabeled avidin can be added to the hybridization complexes, followed by labeled biotin. The result will be a hybridization complex-avidin-labeled biotin "sandwich" which can be detected by an appropriate means.

The results of hybridization assays using the molecular probes of the invention can be made quantitative by preparing standard curves using known amounts of the probes, and then comparing the results to the values of the standard curves.

## EXAMPLES

The present invention may be more readily understood by reference to the following, non-limiting examples.

## PREPARATION OF BIOTINYLATED DNA PROBES

### Example 1 - Preparation of Hybridizable Oligonucleotides

Oligodeoxynucleotides comprising various regions of the nucleotide sequence of the thymidine kinase gene of herpes simplex virus type 1 (HSV-1) [Wagner et al., Proc. Natl. Acad. Sci. USA 78:1441 (1981)] were prepared by the phosphoramidite solid support method of Matteucci et al., J. Am. Chem. Soc. 103:3185 (1981), hereby incorporated by reference, using an Applied Biosystems Inc. Type 380B-02 DNA synthesizer. The nucleotide sequences of these oligodeoxynucleotides, which were maintained affixed to their derivatized controlled porosity glass solid supports following synthesis to facilitate label attachment, were as follows:

1. G G C A T A A G G C A T G C C C A T T G T T (894-915)*
2. C A C A T C G A C C G C C T G G C C A A A C (1153-1174)
3. C A C G G C C T G G G G G G T C A T G C T (1057-1077)*
4. C G A T G A C T T A C T G G C A G G T G C (767-787)
5. C G A C C T G G C G C G C A C G T T T G C C (1602-1623)
6. T G A A C A A A C G A C C C A A C A C C C G (1717-1738)*
7. T C C A G A C C C A C G T C A C C A C C C C (1559-1580)
8. G T T C C T T C C G G T A T T G T C T C C T (1656-1677)*
9. G G C T C C T C A T G T C G G G G G G G A G (933-954)
10. C A G C A T A G C C A G G T C A A G C C G C (1191-1212)*

The numbers in the parentheses to the right of the above oligodeoxynucleotides correspond to the base pair numbering in the thymidine kinase gene disclosed by Wagner et al., supra. The sequence disclosed by Wagner et al. is that of the complement of the transcribed strand. The oligodeoxynucleotides followed by an asterisk are complementary to this strand (i.e., they correspond to the transcribed strand). All sequences above are from 5′ to 3′ (left to right).

Example 2- Biotin Labeling

To produce labeled oligodeoxynucleotides, a two-step process was used. First, a linker molecule was attached directly to the 5′ hydroxyl group of the terminal nucleotide in the oligodeoxynucleotide chain. This linker molecule provides a point for attachment of the polyhydroxyl branching molecules. Second, a branching molecule was attached to the linker molecule to provide two points for further attachment. These attachment points were extended by the addition of two of the linker molecules, and the branching, extension steps were repeated to yield oligodeoxynucleotides covalently coupled at the 5′ positions to a branching network containing four termini. These termini were then converted to amino groups, to which biotin was coupled.

Reagents needed to make the labeled oligodeoxynucleotides were prepared as follows.

All compounds used in the syntheses below were carefully dried prior to their use by coevaporation with pyridine. The compounds were dissolved in pyridine (50 ml), and the solvent was removed under reduced pressure (2 mm Hg). All solvents used were dried according to usual literature methods.

Thin layer chromatography was performed on Kieselgel GO F254 plates (Merck), and the spots were visualized by spraying the plates with phosphomolybdic acid (8% in ethanol) or by dipping the plates in a solution of potassium permanganate (1%) and potassium carbonate (8% in water) and warming them on a hot plate.

Column chromatography was performed on silica gel (Kieselgel 60 Type 7734 70-230 mesh, Merck).

The di-p-methoxytrityl ether of hexathylene was prepared by dissolving 28.23 g (0.1 mole) of hexaethylene glycol (Columbia Organic Chemicals Co.) in 200 ml of dry pyridine. A solution of 16.95 g (0.05 mole) of dimethoxytrityl chloride (Aldrich) in 100 ml of dry pyridine was added dropwise at room temperature under argon. The mixture was stirred at room temperature for 4 hours, after which the solvent was removed under reduced pressure (2 mm Hg.

An oily, yellowish crude residue appeared which was extracted with 300 ml of ethyl acetate, and washed with 200 ml of water followed by two 100-ml volumes of saturated NaCl (brine). The organic layer was dried over anhydrous sodium sulfate and filtered. The solvent was removed and the residue was redissolved in a solution of 30 ml of dichloromethane containing 0.5 ml of pyridine. This mixture was loaded onto a silica gel column (5.5 x 45 cm) and eluted with a mixture of methanol-methylene chloride-pyridine (4:95:1, vol/vol). The product was a yellow oil which had an RF = 0.41 in the above solvents. The yield was 47.2 g (80.7%).

The o-methyl N,N-di-phosphoramidite of di-p-methoxytritylhexaethyleneglycol was prepared by dissolving 5.84 g (10 mmole) of the di-p-methoxytrityl ether of hexaethyleneglycol in 30 ml of dry methylene chloride under argon. Dry N,N-diisopropylethylamine (Aldrich; 5.16 g, 40 mmole) was added followed by the dropwise addition of 1.97 g (10 mmole) of N,N-diisopropylmethylchlorophosphoramidite (Aldrich).

The solution was stirred at room temperature for 30 minutes, diluted with 300 ml of ethyl acetate, and washed with saturated $NaHCO_3$ (100 ml) and with brine (100 ml). The organic layer was dried over anyhdrous $Na_2SO_4$, and the solvent was removed under reduced pressure (15 mm Hg).

Further drying was achieved by dissolving the residue in toluene (2 x 100 ml) and evaporating the solvent, and by lyophilization in dry benzene. The product (7.21 g, 96.7%) was obtained as a yellow oil having an RF = 0.63 in methanol-methylene chloride (5:95). The product was characterized by its mass spectrum and by its nmr spectrum, which is shown in Fig. 2.

1,4,7-Heptanetriol was prepared by suspending 4.2 g of lithium aluminum hydride (Aldrich) in 150 ml of dry ether in a 3-necked flask equipped with a magnetic stirrer, a dropping funnel, a reflux condenser and an inlet for argon. Dimethyl 4-oxoheptanoate (Aldrich; 9.9 g, 49. 5 mmole ) in 150 ml of dry ether was added dropwise at such a rate that a mild reflux was maintained. After the addition was complete, the solution was refluxed for one hour and cooled to room temperature. Twenty ml of ethyl acetate followed by 15 ml of water were then added.

The reaction mixture was poured into 500 ml of ice water, and 100 ml of 10% sulfuric acid were added. The layers were separated, and the organic layer was washed with 100 ml of water, twice with 150-ml volumes of saturated sodium bicarbonate and once with 100 ml of brine. The layer was filtered and the solvent was removed under reduced pressure.

The product was obtained as an oily material in a yield of 7.03 g (97%) and was characterized by its spectral properties.

The 1,7-di-p-methoxytrityl ether of 1,4,7-heptanetriol was prepared by dissolving 7 g (47.3 mmole) of 1,4,7-heptanetriol in 200 ml of dry pyridine. Dimethoxytrityl chloride (Aldrich; 32.05 g, 94.6 mmole) in 150 ml of dry pyridine was added dropwise, and the reaction mixture was stirred for 1 hour at room temperature. The pyridine was removed under low pressure (2 mm Hg), and the residue was dissolved in

300 ml of ethyl acetate, washed with 100 ml of saturated $NaHCO_3$ followed by 100 ml of brine, and dried over anyhdrous $Na_2SO_4$. The mixture was filtered, and the solvent was removed under reduced pressure (15 mm Hg).

The residue was pruified by column chromatography in silica gel, using ethyl ethyl acetate-hexane-pyridine (1:3:0.0025, vol/vol) as the eluant. Fractions of about 200 ml were collected, and the product eluted in fractions 8-10. Following solvent removal, a yield of 7.3 g (25%) of a product having an RF = 0.48 in ethyl acetate-hexane (10:25, vol/vol) TLC was obtained. The product was characterized by nmr and mass spectral analysis.

The O-methyl N,N-diisopropylphosphoramidite of the 1,7-di-p-methoxytrityl ether of 1,4,7-heptanetriol was prepared by dissolving 8.4 g (11.1 mmole) of 1,7-di-(p-dimethoxytrityl)-4-heptanetriol in 30 ml of dry methylene chloride under argon and then adding 8 ml (45 mmole) of dry diisopropylethylamine followed by 2.2 g (11.1 mmole) of chloro N,N-diisopropylmethylphosphoramidite. The mixture was stirred at room temperature for 30 minutes.

The solution was diluted with 300 ml of ethyl acetate and washed with 10 ml of saturated sodium bicarbonate and 10 ml of brine. The organic layer was dried over anhydrous $Na_2SO_4$ and filtered, and the solvent was removed under reduced pressure (15 mm Hg) to yield an oily residue. This residue was further dried by coevaporation twice in 100-ml volumes of dry toluene and lyophilization in dry benzene. The product, which was confirmed by mass spectral analysis, had an RF = 0.75 in ethyl acetate-hexane (10:25, vol/vol) and an nmr spectrum shown in Fig. 3.

6-Trifluoroacetamidohexanol was prepared by dissolving 5.86 g (50 mmole) of 6-aminohexanol (Aldrich) in 100 ml of pyridine. Triethylamine (7.7 ml) was added, and the solution was cooled in an ice bath. Trifluoroacetic anhydride (Aldrich; 7.25 ml) was added dropwise, and the solution was allowed to warm to room temperature and stirred for 1 hour. The solvent was removed under reduced pressure (40°C, 15 mm Hg). The residue was dissolved in 100 ml of 10% aqueous pyridine, and the solution was stirred at room temperature for 10 minutes and then extracted with 100 ml of chloroform. The organic layer was washed with 50 ml of water and dried over anhydrous magnesium sulfate, and the solvent was removed under reduced pressure. The residue was further dried by evaporating it twice from 20-ml portions of toluene. The yield was 6.1 g (57%), and the structure was confirmed by mass spectral and nmr analysis.

The o-methyl N,N-diisopropylphosphoramidite of 6-trifluoracetamidohexanol was prepared by dissolving 2.13 g (10 mmole) of 6-trifluoroacetamidohexanol in 30 ml of dichloromethane. Dry N,N-diisopropylethylamine (Aldrich; 5.16 g, 40 mmole) was added, followed by 1.97 g (10 mmole) of chloro N,N-diisopropylmethylphosphoramidite, and the solution was stirred at room temperature under argon for 30 minutes.

Three-hundred ml of ethyl acetate were added and the solution was washed with 100 ml of saturated $NaHCO_3$ and 100 ml of brine, and dried over anhydrous $Na_2SO_4$. The solution was filtered and the solvent was removed under reduced pressure (15 mm Hg). The oily residue was further dried by coevaporation twice in 100-ml portions of dry toluene and lyophilization in dry benzene. The yield was 3.5 g (93%), and the product was characterized by mass spectral analysis and had an nmr spectrum shown in Fig. 4.

Using the above reagents, biotinylated oligodeoxynucleotide probes containing 4 biotin reporter groups were prepared by a procedure which entailed the addition in sequence to the above immobilized oligodeoxynucleotides of:

(1) a hexaethyleneglycol linker molecule,
(2) a heptanetriol branching molecule,
(3) two hexaethyleneglycol linker molecules,
(4) two heptanetriol branching molecules,
(5) four hexaethyleneglycol linker molecules,
(6) four aminohexaethyleneglycol linker molecules, and
(7) four biotin reporter groups.

The specific manner in which these syntheses were carried out follows.

Following the last cycle in the synthesis of each of the above immobilized oligodeoxynucleotides, 0.075 g of dimethoxytrityl-hexaethyleneglycol-(o-methyl N,N-diisopropyl)phosphoramidite dissolved in 0.6 ml of dry $CH_3CN$ was added, and the cycle was carried out as described for the oligonucleotide synthetic steps by Matteucci et al., supra. The coupling time was 6 minutes.

Briefly, each cycle as described by Matteucci et al. involved the following steps:

1. Twenty-five milligrams of tetrazole in 0.6 ml of dry $CH_3CN$ were added at the start of each cycle followed by the addition of the phosphoramidite of the next linker or branching molecule to be coupled, for 6 minutes.

2. The system was washed with 5 ml of tetrahydrofuran:

3. A solution of 1 ml of acetic anhydride-tetrahydrofuran-lutidine (1:8:2, vol/vol) was added with 2 ml of 100 mg/ml dimethylaminopyridine in tetrahydrofuran, for 2 minutes;

4. The system was washed with 5 ml of tetrahydrofuran;

5. Two milliliters of a solution of 0.1 M $I_2$ in tetrahydrofuran-lutidine-$H_2O$ (2:2:0.5, vol/vol) were added for 2 minutes;

6. The system was washed with 5 ml of $CH_3CN$; and

7. Two milliliters of a 2% solution of trichloroacetic acid in dichloromethane were added for 30 seconds.

A heptanetriol branching molecule was added to the hexaethyleneglycol linker molecule by reacting 0.075 g of 1,7-di-(o-dimethoxytrityl)-4-(o-methyl N,N-diisopropyl) phosphoramidite dissolved in 0.6 ml of dry $CH_3CN$ in the same fashion. The coupling time for this step was 10 minutes.

Two hexaethyleneglycol linker molecules were added to the heptanetriol branching molecules by adding 0.150 g of dimethoxytrityl-hexaethyleneglycol-(o-methyl N,N-diisopropyl)phosphoramidite in 0.8 ml of dry $CH_3CN$ for a coupling time of 10 minutes.

Two heptanetriol branching molecules were added to the hexaethylene glycol linker molecules by adding 0.20 g of 1,7-di-(o-dimethoxytrityl)-4-(o-methyl N,N-diisopropyl) phosphoramidite in 1 ml of dry $CH_3CN$ for a coupling time of 15 minutes.

Four hexaethyleneglycol linker molecules were added to the two heptanetriol branching molecules by adding 0.20 g of dimethoxytrityl-hexaethyleneglycol-(o-methyl N,N-diisopropyl)phosphoramidite in 1 ml of dry $CH_3CN$ for a coupling time of 15 minutes.

Four aminohexanol linker molecules were added to extend the hexaethyleneglycol linker molecules by the following procedure. Two-tenths g of 6-trifluoroacetamidohexanol-(o-methyl N,N-diisopropyl) phosphoramidite was added in 1.2 ml of dry $CH_3CN$ for a coupling time of 20 minutes. The immobilized, modified oligonucleotide was then cleaved from the solid support and all blocking groups were removed as follows. Two ml of a solution of thiophenol-triethylamine-dioxane (1:2:2, vol/vol) were added and allowed to react for 75 minutes at room temperature. The support was washed with 30 ml of tetrahydrofuran and then treated with 2 ml of concentrated (30%) ammonium hydroxide at 37°C for 2 hours. Then, the supernatant fluid was transferred to a sealed vial and heated at 50°C for 16 hours. The ammonium hydroxide was evaporated to dryness, and the residue was redissolved in 1 ml of $H_2O$, washed 3 times with 1-ml portions of n-butanol and evaporated to dryness.

The modified oligodeoxynucleotides were purified by preparative polyacrylamide gel electrophoresis in 3 mm thick x 30 cm long 12% gels, using tris-borate buffer (pH 8.0) containing 7 M urea. Approximately 100.D units (measured at 260 nm) of the crude material in 30 µl of 80% formamide were loaded into 2.5 cm wide wells, and electrophoresis was carried out at 16 volts/cm.

Following electrophoresis, the gels were visualized by placement over a fluorescent TLC plate (Sigma Chemical Co.) and illumination with long wavelength ultra-violet light. The oligodeoxynucleotide bands absorbed intensely, and the bands were excised from the gels and extracted with 0.5 M ammonium acetate, 10 mM $MgCl_2$, 0.1% sodium dodecyl sulfate and 0.1 mM ethylenediaminetetracetic acid (EDTA). The extracts were washed with 1 ml of n-butanol, and the modified oligodeoxynucleotides were desalted in Sephadex® G-50/40 columns (2.5 x 45 cm, Pharmacia Fine Chemicals) using double distilled water. Recoveries generally ranged from 10-20%, based on the $100.D._{260}$ units of total material purified.

The modified oligodeoxynucleotides were biotinylated by lyophlyzing $1.0.O.D._{260}$ of the purified material to dryness, and redissolving the material in 100 µl of 0.1 M sodium bicarbonate buffer, pH 8.5. One hundred microliters of 0.1 M N-hydroxysuccinimidobiotin (Sigma Chemical Co.) in dimethylsulfoxide were added, and the mixture was kept at 4°C for 16 hours. The unreacted biotin was then removed by chromatography in Sephadex® G-50/40 (1 x 10 cm) using double-distilled water as the eluant. One-ml fractions were collected and monitored spectrophotometrically at 260 nm. Fractions containing the biotinylated oligodeoxynucleotides were pooled and lyophlyzed to dryness for storage.

## DNA HYBRIDIZATION

Example 3-Hybridization of Biotinylated Oligodeoxynucleotides to HSV-1 Thymidine Kinase Gene

To demonstrate the use of the biotinylated DNA probes of the invention in a hybridization assay, the quadruplicately-biotinylated oligodeoxynucleotides corresponding to various regions of the HSV-1 thymidine kinase gene described in Example 1 were tested against target DNA containing the thymidine kinase gene. This DNA was contained in a plasmid designated HSV-106, which was obtained from Bethesda Research Laboratories, Gaithersburg, MD (Catalog No. 5365 SA).

The target DNA, which was a double-stranded DNA, was dissolved in 1 M ammonium acetate buffer, pH 7.5, at a concentration of 20 µg/ml. This DNA solution was boiled for 5 minutes to effect strand separation, and then quickly cooled in an ice-water bath to prevent reannealing. The DNA solution was diluted with the ammonium acetate buffer to a concentration of 0.1 µg/ml, and serial two-fold dilutions were made of this solution for assay.

Fifty-microliter aliquots of the DNA solutions were added to the wells of 96-well Dynatech Immulon II microliter plates. The plates were sealed and incubated for 90 minutes at 37° C to bind the DNA to the plates. After the incubation, the wells were washed at room temperature twice with 200 µl aliquots of 0.3 M NaCl and 0.03 M sodium citrate, pH 7.0, and once with 200 µl of the same buffer containing 0.1% Triton X-100.

The biotinylated probes were added to a stock hybridization mixture at a concentration of 50 ng/ml, either singly or in mixtures containing a number of the probes. This concentration of each probe was sufficient to saturate all available binding sites under the conditions of hybridzation employed. Where a mixture of the probes was used, the concentration of each probe was 50 ng/ml. The hybridization mixture buffer contained 30% deionized formamide (vol/vol), 0.54 M NaCl, 0.03 M sodium phosphate (pH 7.4), 0.003 M EDTA, 5% dextran sulfate (w/vol) and 0.1% Triton X-100. The deionized formamide had been prepared by adding 1 g of Bio-Rad AG 501-X8(D) 20-50 mesh mixed bed resin to 50 ml of formamide (Sigma Chemical Co.) and mixing for 30 minutes at room temperature. The formamide was then filtered twice through Whatman No. 1 filter paper. The dextran sulfate (500.000 molecular weight) was from Sigma.

One hundred-microliter aliquots of the hybridization mixture containing one or more of the biotinylated probes were pipetted into the wells of the microtiter plates and the plates were sealed and incubated for 1 hour at room temperature to permit hybridization to occur. The wells were then washed twice with 0.3 M NaCl, 0.003 M sodium citrate and 0.1% Triton X-100 (pH 7.0) at room temperature and four times with 0.03 M NaCl, 0.003 M sodium citrate and 0.1% Triton X-100 (pH 7.0) at 42° C. All of the washes were quickly carried out without incubation.

The wells were treated or 30 minutes at room temperature with 200-µl aliquots of 10 mM $NaPO_4$, pH 7.4, 0.5 M NaCl, 2% BSA, 0.1% Triton X-100 and 5 mM EDTA (blocking solution), to prevent subsequent nonspecific binding of the reagent used to detect the biotinylated probe-target DNA hybridization complexes formed. The blocking solution was removed from the wells, and 50 ul aliquots of a solution containing horseradish peroxidase-labeled avidin (Vector Laboratories, Burlingame, CA; catalog No. PK-4000) were added to each well. The peroxidase-labeled avidin was diluted in PBS, 0.1% Triton X-100 according to the manufacturer's instructions.

The plates were incubated for 30 minutes at room temperature, after which the wells were washed 4 times with 200-µ aliquots of 10 mM $NaPO_4$, pH 7.4, 0.5 M NaCl, 0.1% BSA, 0.1% Triton X-100 and 5 mM EDTA and then once with PBS containing 1 mM EDTA.

The target DNA-biotinylated probe-labeled avidin complexes in the wells were detected by adding to each well 150 µl of a substrate reaction mixture containing 1.6 mg/ml disodium o-phenylenediamine (Sigma) and 0.0125% $H_2O_2$ in 0.1 M $Na_2HPO_4$ buffer, adjusted to pH 6.0 by the addition of 0.05 M citric acid. the plates were incubated for 30 minutes at room temperature in the dark, when the reaction was stopped by the addition to each well of 50 µl of 4 N $H_2SO_4$. The contents of the wells were then read at 490 nm in an Intermed Immuno Reader NJ-2000 spectrophotometric plate reader, with the results shown in Table I.

TABLE 1

Hybridization of Biotinylated Probes to HSV-1 DNA

Absorbance ($O.D._{490}$)

| | Amount of Target DNA[a] Added (ng) | | | | | |
|---|---|---|---|---|---|---|
| Probe No(s). | 5 | 2.5 | 1.25 | 0.625 | 0.3125 | Control[b] |
| 1 | 0.147 | 0.083 | 0.053 | 0.035 | 0.026 | 0.009 |
| 2 | 0.207 | 0.119 | 0.083 | 0.053 | 0.041 | 0.015 |
| 3 | 0.324 | 0.172 | 0.105 | 0.060 | 0.033 | 0.013 |
| 4 | 0.121 | 0.059 | 0.037 | 0.020 | 0.012 | 0.000 |
| 5 | 0.167 | 0.093 | 0.059 | 0.029 | 0.017 | 0.012 |
| 6 | 0.203 | 0.113 | 0.075 | 0.048 | 0.041 | 0.008 |
| 7 | 0.143 | 0.073 | 0.043 | 0.025 | 0.021 | 0.005 |
| 8 | 0.166 | 0.081 | 0.050 | 0.031 | 0.023 | 0.002 |
| 9 | 0.171 | 0.089 | 0.051 | 0.033 | 0.030 | 0.004 |
| 10 | 0.193 | 0.113 | 0.079 | 0.048 | 0.036 | 0.006 |
| 1-5[c] | 0.851 | 0.474 | 0.269 | 0.149 | 0.091 | 0.031 |
| 1-10[c] | 0.191 | 0.682 | 0.384 | 0.201 | 0.129 | 0.028 |

a The amount of the target DNA shown is the amount of plasmid HSV-106 that was originally added to the wells for binding.

b Control means 5 ng of an unrelated plasmid DNA which has no nucleotide sequence in common with the HSV-1 thymidine kinase gene.

c Probes specified in these rows are mixtures of the indicated probes.

The data of Table 1 show that the binding of all of the probes to the target DNA could readily be detected, even when as little as 0.3125 ng of DNA was originally added to the wells. The data also show that the strength of the signal observed was greatly increased when mixtures of probes complementary to different regions along the target DNA were used.

Many modifications and variations of this invention may be made without departing from its spirit and scope, as will become apparent to those skilled in the art. The specific embodiments described herein are offered by way of example only, and the invention is limited only by the terms of the appended claims.

## Claims

1. A labelled, single-stranded DNA molecule, comprising:

(a) an oligonucleotide complementary to a specific RNA or DNA sequence which is capable of specifically hybridizing thereto; and

(b) a labeling array covalently coupled to the 5′ terminal hydroxy group of the oligonucleotide which comprises in order from such hydroxyl group

(i) a first linker molecule of the formula

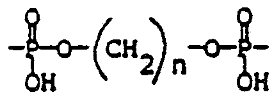 or 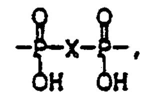

where n = a number from 2 to 10 and X represents a polyethylene glycol chain having from 1 to 30 ethylene glycol units;

(ii) a polyhydroxyl branching molecule having from 3 to 5 hydroxyl groups, one of which is covalently coupled to the first linker molecule through its distal phosphate group; and

(iii) second linker molecules of the formula

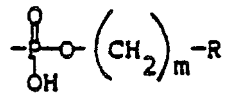 or 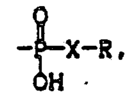

where m = a number from 2 to 10, X represents a polyethylene glycol chain having from 1 to 30 ethylene glycol units, and R is selected from the group consisting of a reporter group; one or more additional first linker molecules terminated by a reporter group; or one or more labeling arrays comprising such first linker molecules, polyhydroxyl branching molecules and second linker molecules, the ends distal to the oligonucleotide of which are terminated by reporter groups;

which second linker molecules are covalently coupled by the phosphate groups to the other hydroxyl groups of such branching molecule.

2. The labeled DNA molecule of claim 1 in which the reporter group is a radioisotope.

3. The labeled DNA molecule of claim 1 in which the reporter group is a fluorescent molecule.

4. The labeled DNA molecule of claim 1 in which the reporter group is electron opaque.

5. The labeled DNA molecule of claim 1 in which the reporter group is an enzyme.

6. The labeled DNA molecule of claim 1 in which the reporter group is an unlabeled, indirectly detectable first member of a specific binding pair, which first member is detectable by addition to a hybridization complex containing such a modified DNA molecule of a labeled, second member of such binding pair, or an unlabeled second member of such binding pair followed by a labeled molecule specific for such second member.

7. The labeled DNA molecule of claim 6 in which the reporter group is unlabeled biotin, which biotin is detected by addition to a hybridization complex containing such a modified DNA molecule of labeled avidin.

8. The labeled DNA molecule of claim 6 in which the reporter group is unlabeled biotin, which biotin is detected by addition to a hybridization complex containing such a modified DNA molecule of unlabeled avidin, followed by labeled biotin.

9. The labeled DNA molecule of claim 6 in which the reporter group is an unlabeled hapten molecule, which hapten molecule is detected by addition to a hybridization complex containing such a modified DNA molecule of labeled antibodies against the hapten molecule.

10. The labeled DNA molecule of claim 6 in which the reporter group is an unlabeled hapten molecule, which hapten molecule is detected by addition to a hybridization complex containing such a modified DNA molecule of unlabeled first antibodies against the hapten molecule, followed by labeled antibodies against an immunoglobulin chaim of such first antibodies.

11. A method for detecting a specific RNA or DNA sequence in a biological sample, comprising:

(a) contacting a biological sample suspected to contain a specific RNA or DNA sequence under conditions which promote specific hydridization of complementary nucleotide sequences with a labeled single-stranded DNA molecule, comprising:

(i) an oligonucleotide complementary to the specific RNA or DNA sequence which is capable of specifically hydridizing thereto; and

(ii) a labeling array covalently coupled to the 5′ terminal hydroxyl group of the oligonucleotide which comprises in order from such hydroxyl group

(A) a first linker molecule of the formula

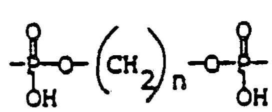 or 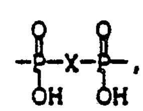

where n = a number from 2 to 10 and X represents a polyethylene glycol chain having from 1 to 30 ethylene glycol units;

(B) a polyhydroxyl branching molecule having from 3 to 5 hydroxyl groups, one of which is covalently coupled to the first linker molecule through its distal phosphate group; and

(C) second linker molecules of the formula

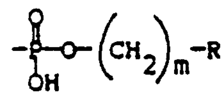 or 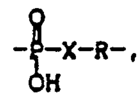

where m = a number from 2 to 10, X represents a polyethylene glycol chain having from 1 to 30 ethylene glycol units, and R is selected from the group consisting of a reporter group; one or more additional first linker molecules terminated by a reporter group; or one or more labeling arrays comprising such first linker molecules, polyhydroxyl branching molecules and second linker molecules, the ends distal to the oligonucleotide of which are terminated by reporter groups;

which second linker molecules are covalently coupled by the phosphate groups to the other hydroxyl groups of such branching molecule; and

(b) detecting any hydridization which occurs between such labeled DNA molecule and specific RNA or DNA sequence in the biological sample.

-- 12. A modified, single-stranded DNA molecule, comprising:

(a) an oligonucleotide complementary to a specific RNA or DNA sequence which is capable of specifically hybridizing thereto; and

(b) an array covalently coupled to the 5′ terminal hydroxy group of the oligonucleotide which comprises in order from such hydroxyl group

(i) a first linker molecule of the formula

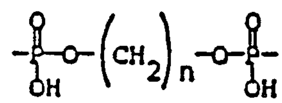 or 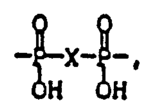

where n = a number from 2 to 10 and X represents a polyethylene glycol chain having from 1 to 30 ethylene glycol units;

(ii) a polyhydroxyl branching molecule having from 3 to 5 hydroxyl groups, one of which is covalently coupled to the first linker molecule through its distal phosphate group; and

(iii) second linker molecules of the formula

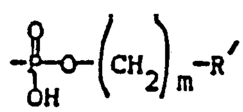 or 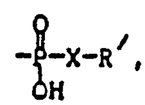

where m = a number from 2 to 10, X represents a polyethylene glycol chain having from 1 to 30 ethylene glycol units, and R is selected from the group consisting of:

A. an end group selected from the group consisting of carboxyl, sulfhydryl, maleimide, isocyanate, isothiocyanate and amino groups;

B. one or more additional first linker molecules terminated by end groups selected from the group consisting of carboxyl, sulfhydryl, maleimide, isocyanate, isothiocyanate and amino groups; and

C. one or more arrays comprising such first linker molecules, polyhydroxyl branching molecules and second linker molecules covalently coupled by the phosphate groups to the other hydroxyl groups of such branching molecules, the ends distal to the oligonucleotide of which are terminated by end groups selected from the group consisting of carboxyl, sulfhydryl, maleimide, isocyanate, isothiocyanate and amino groups;

which modified DNA molecule is capable of being covalently coupled at the termini distal to the complementary oligonucleotide to one or more reporter groups, to produce a labeled DNA molecule. --

FIG.1

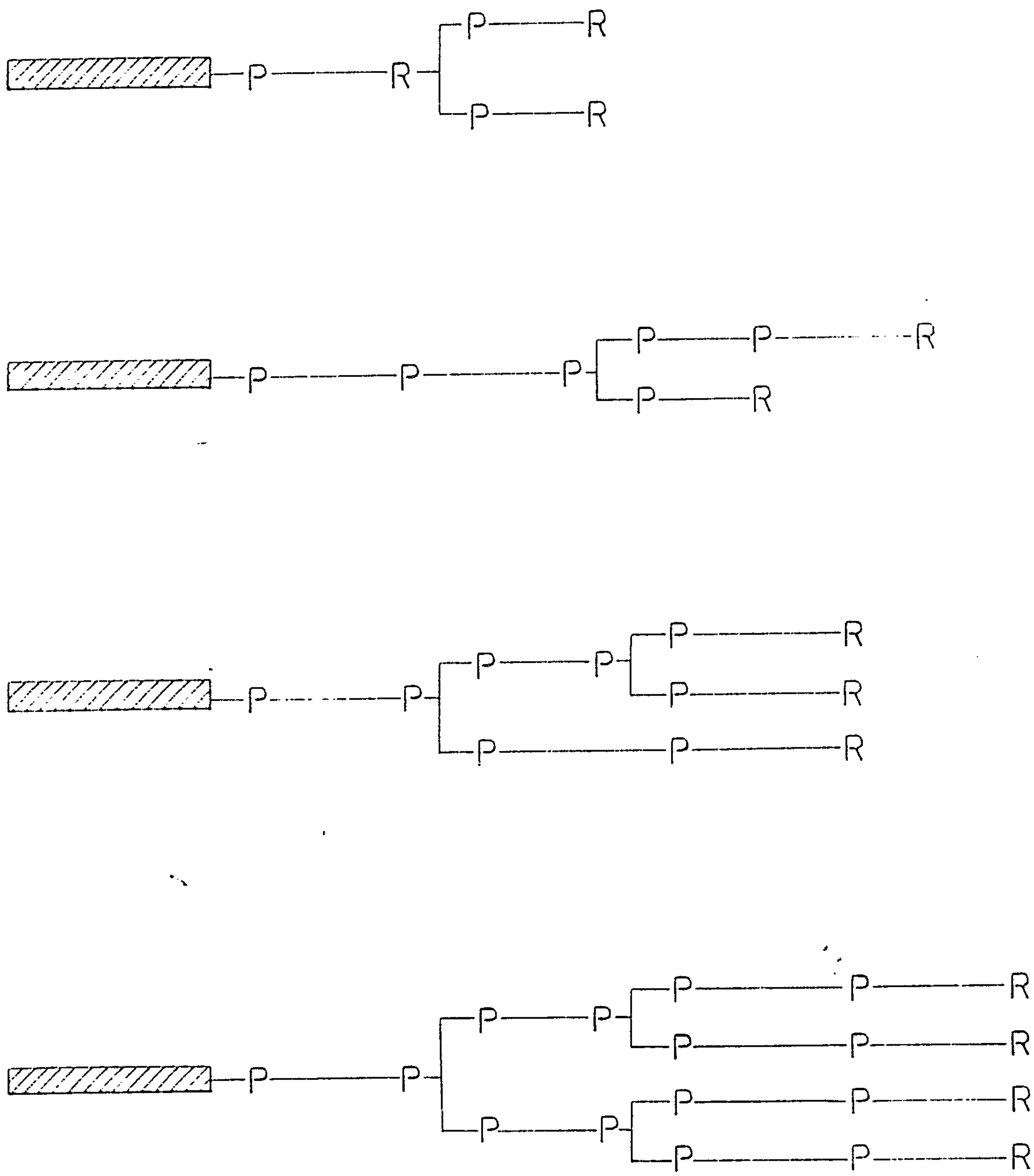

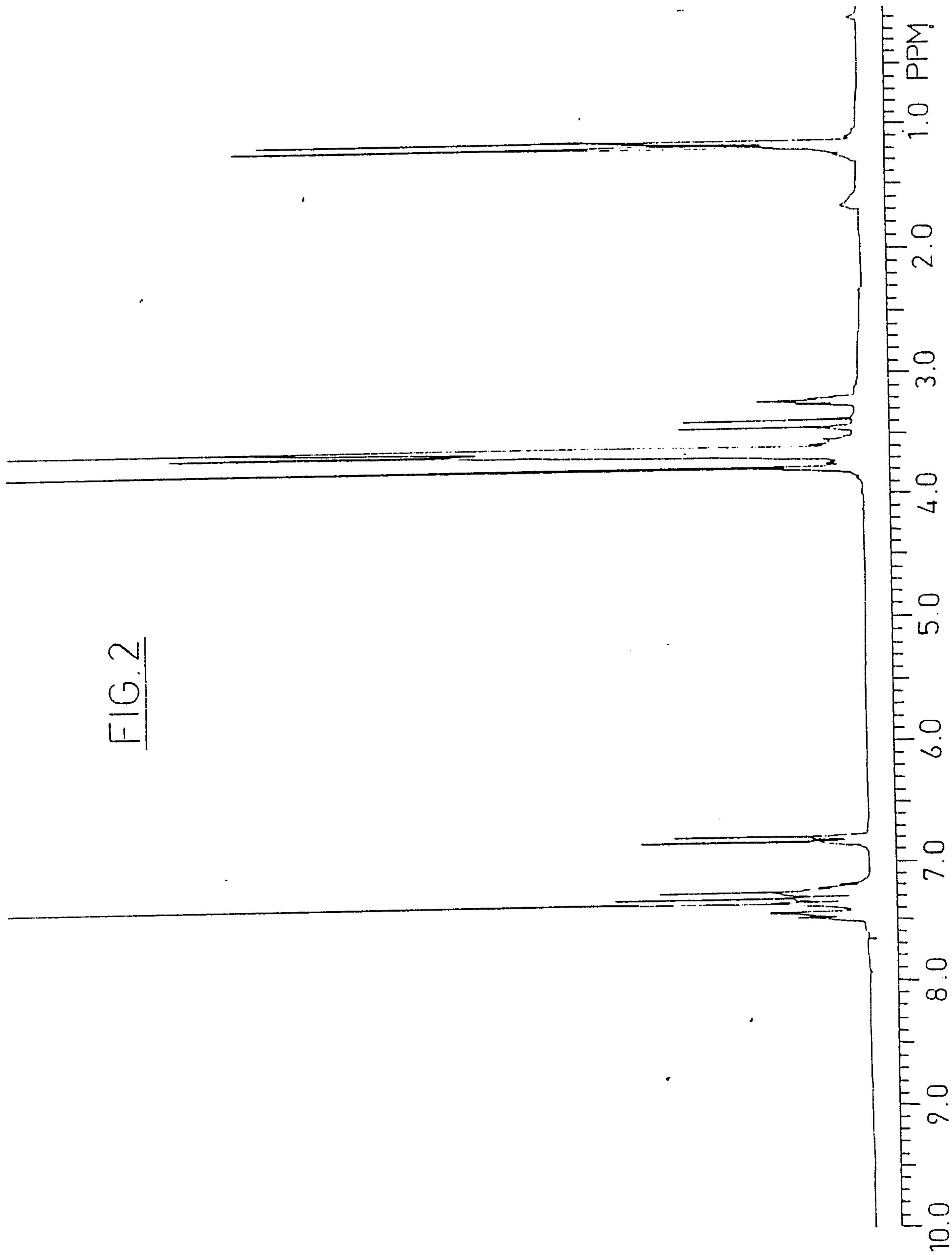


FIG. 2

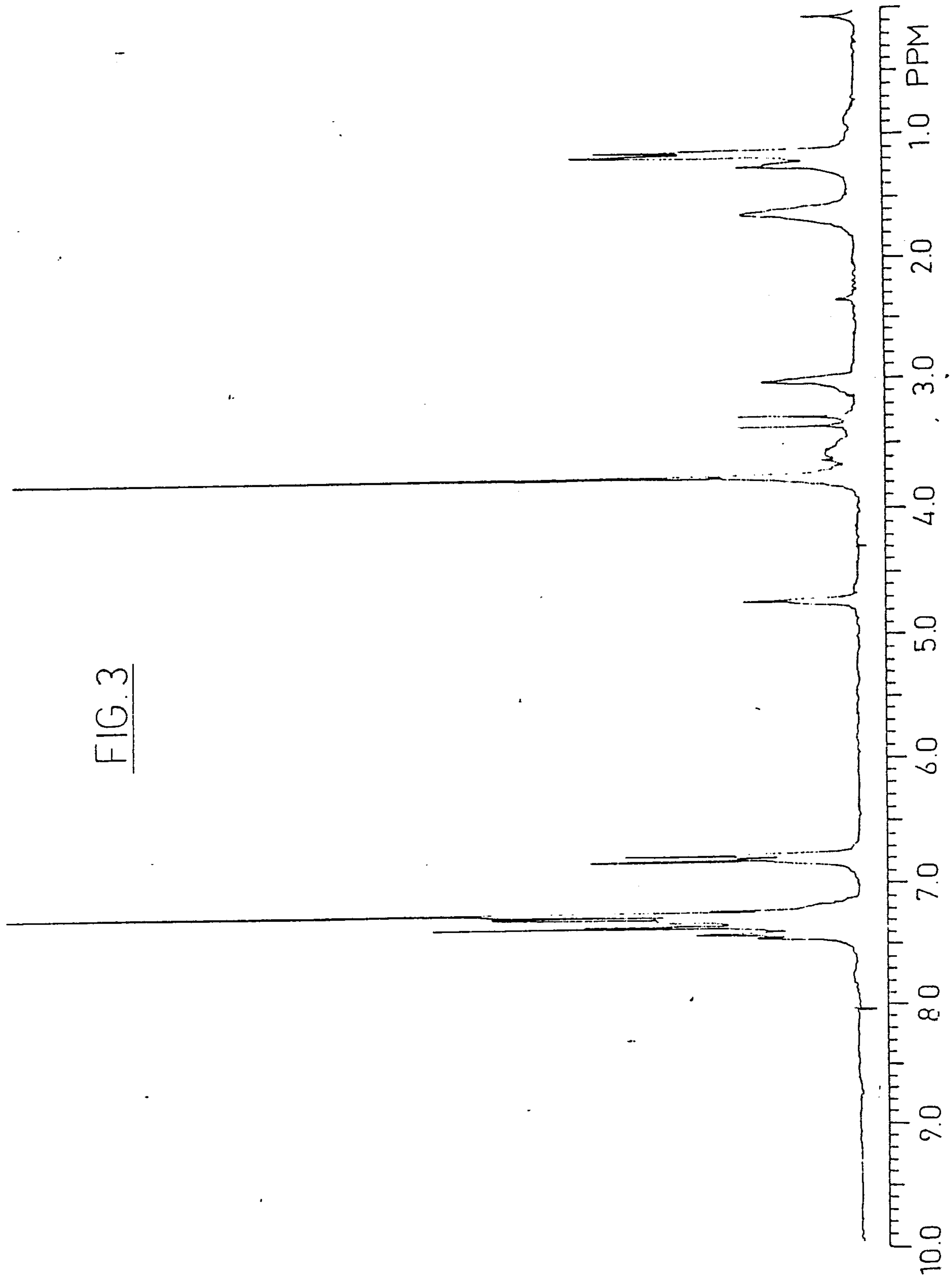


FIG.3

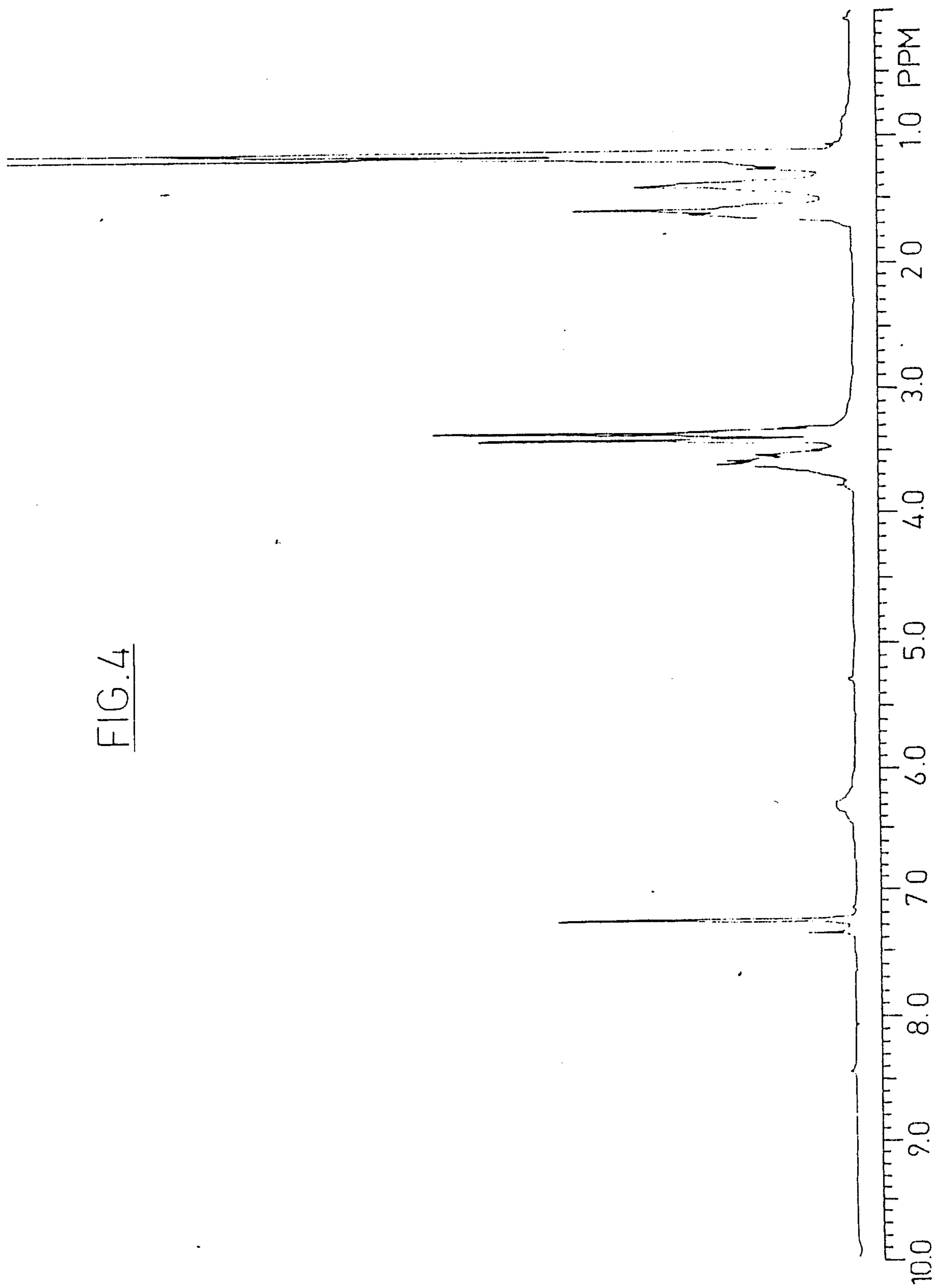


FIG.4

European Patent Office

# EUROPEAN SEARCH REPORT

Application Number

EP 88 30 3847

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | WO-A-8 605 519 (J. SUMMERTON)<br>* Page 2, line 27 - page 3, line 9; page 14, lines 10-28; page 42, lines 18-28; page 46, lines 18-28; page 50, lines 1-19; page 51, lines 5-23; page 121, line 3 - page 128, line 17 *<br>--- | 1-11 | C 12 Q 1/68 |
| X | WO-A-8 502 628 (HRI RESEARCH CORP.)<br>* Page 3, line 21 - page 5, line 18; page 8, line 28 - page 11, line 35; page 14, line 19 - page 15, line 11; page 16, lines 1-23; page 29, line 2 - page 35, line 2 *<br>--- | 1-11 | |
| X | EP-A-0 138 357 (INTEGRATED GENETICS)<br>* Page 1, line 30 - page 2, line 29; page 19, line 30 - page 22, line 9; claims 1-10 *<br>--- | 1-11 | |
| X | EP-A-0 151 001 (E.I. DU PONT DE NEMOURS & CO.)<br>* Page 3, lines 8-35; page 5, line 21 - page 9, line 10; page 11, lines 1-23; page 33, line 1 - page 36, line 9 *<br>--- | 1,3-12 | TECHNICAL FIELDS SEARCHED (Int. Cl.4) |
| X | DNA, vol. 4, no. 1, 1985, page 93, US; J.R. RUTH et al.: "Linker arm nucleotide analogs useful in oligonucleotide synthesis"<br>* Whole article *<br>--- | 1-11 | C 12 Q 1/00<br>C 12 N 15/00 |
| Y | EP-A-0 164 586 (MOLECULAR DIAGNOSTICS)<br>* Page 2, line 20 - page 4, line 20; page 10, line 3 - page 11, line 19 *<br>--- -/- | 1-12 | |

The present search report has been drawn up for all claims

| Place of search | Date of completion of the search | Examiner |
|---|---|---|
| THE HAGUE | 14-09-1988 | VAN BOHEMEN C.G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent Office

# EUROPEAN SEARCH REPORT

Application Number

EP 88 30 3847

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | JOURNAL OF MOLECULAR BIOLOGY, vol. 133, pages 319-338, Academic Press INC., London, GB; T. NILSEN et al.: "Unusual base-pairing of newly synthesized DNA in HeLa cells" * Page 322, line 47 - page 323, line 5; page 323, line 5; page 334, figure 11; page 336, figure 12; page 336, lines 1-14 * ----- | 1-12 | |
| | | | TECHNICAL FIELDS SEARCHED (Int. Cl.4) |

The present search report has been drawn up for all claims

| Place of search | Date of completion of the search | Examiner |
|---|---|---|
| THE HAGUE | 14-09-1988 | VAN BOHEMEN C.G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)